# EUROPEAN PATENT SPECIFICATION

(11) **EP 2 327 445 B1**
(45) Date of publication and mention of the grant of the patent: **10.10.2018**
(21) Application number: 10177808.2
(22) Date of filing: 01.06.2005
(51) Int. Cl.: A61M 39/02

(54) **A surgically implantable injection port having an improved fastener**
Selbsbefestigende subkutane Injektionsöffnung mit integrierten, bewegbaren Befestigungsteilen
Orifice d'injection sous-cutanée auto-attachant, avec des membres de retenue intégrés

(30) Priority: 01.06.2004 US 858614
(43) Date of publication of application: 01.06.2011
(62) Divisional of application: 05253363.5
(73) Proprietor: ETHICON ENDO-SURGERY, INC., Cincinnati, Ohio 45242 (US)
(72) Inventor: Comlon, Sean P., Loveland, OH 45140 (US); Uth, Joshua R., Mason, OH 45040 (US); Chen, How-Lun, Cincinnati, OH 45242 (US)
(74) Representative: Tunstall, Christopher Stephen

(56) References cited:
- EP-A- 1 346 753
- EP-A- 1 488 824
- WO-A1-2005/072627
- WO-A2-2005/037055
- DE-A1- 19 745 654
- US-A- 5 207 644
- US-A- 5 976 159
- US-A1- 2003 004 520

## Description

### Field of the Invention

The present invention has application in conventional endoscopic and open surgical instrumentation as well as application in robotic-assisted surgery. The present invention has even further relation to adjustable surgically implantable bands, such as gastric bands for the treatment of obesity.

### Background of the Invention

The percentage of the world's population suffering from morbid obesity is steadily increasing. Severely obese persons are susceptible to increased risk of heart disease, stroke, diabetes, pulmonary disease, and accidents. Because of the effect of morbid obesity to the life of the patient, methods of treating morbid obesity are being researched.

Numerous non-operative therapies for morbid obesity have been tried with virtually no permanent success. Dietary counseling, behavior modification, wiring a patient's jaws shut, and pharmacological methods have all been tried, and failed to correct the condition. Mechanical apparatuses for insertion into the body through non-surgical means, such as the use of gastric balloons to fill the stomach have also been employed in the treatment of the condition. Such devices cannot be employed over a long term, however, as they often cause severe irritation, necessitating their periodic removal and hence interruption of treatment. Thus, the medical community has evolved surgical approaches for treatment of morbid obesity.

Most surgical procedures for treatment of morbid obesity may generally be classified as either being directed toward the prevention of absorption of food (malabsorption), or restriction of stomach to make the patient feel full (gastric restriction) The most common malabsorption and gastric restriction technique is the gastric bypass. In variations of this technique, the stomach is horizontally divided into two isolated pouches, with the upper pouch having a small food capacity. The upper pouch is connected to the small intestine, or jejunum, through a small stoma, which restricts the processing of food by the greatly reduced useable stomach. Since food bypass much of the intestines, the amount of absorption of food is greatly reduced.

There are many disadvantages to the above procedure. Typically the above mentioned procedure is performed in an open surgical environment. Current minimally invasive techniques are difficult for surgeons to master, and have many additional drawbacks. Also, there is a high level of patient uneasiness with the idea of such a drastic procedure which is not easily reversible. In addition, all malabsorption techniques carry ongoing risks and side effects to the patient, including malnutrition and dumping syndrome.

Consequently, many patients and physicians prefer to undergo a gastric restriction procedure for the treatment of morbid obesity. One of the most common procedures involves the implantation of an adjustable gastric band. Examples of an adjustable gastric band can be found in U.S. Patents 4,592,339 issued to Kuzmak; RE 36176 issued to Kuzmak; 5,226,429 issued to Kuzmak; 6,102,922 issued to Jacobson and 5,601,604 issued to Vincent. In accordance with current practice, a gastric band is operatively placed to encircle the stomach. This divides the stomach into two parts with a stoma in-between. An upper portion, or a pouch, which is relatively small, and a lower portion which is relatively large. The small partitioned portion of the stomach effectively becomes the patients new stomach, requiring very little food to make the patient feel full.

Once positioned around the stomach, the ends of the gastric band are fastened to one another and the band is held securely in place by folding a portion of the gastric wall over the band and closing the folded tissue with sutures placed therethrough thereby preventing the band from slipping and the encircled stoma from expanding. Gastric bands typically include a flexible substantially non-extensible portion having an expandable, inflatable portion attached thereto. The inflatable portion is in fluid communication with a remote injection site, or port. Injection or removal of an inflation fluid into or from the interior of the inflatable portion is used to adjust the size of the stoma either during or following implantation. By enlarging the stoma, the patient can eat more food without feeling as full, but will not lose weight as fast. By reducing the size of the stoma, the opposite happens. Physicians regularly adjust the size of stoma to adjust the rate of weight loss.

For most fluid injection ports for the above described bands are attached underneath the skin to the fascia of a patient. Such ports are often provided with suture holes and the port is sutured or screwed to the tissue. However, alternative means of attaching the port to the patient, such as using integral hooks, can be used as well. Such other means for attaching the port to a patient are described in commonly assigned and copending U.S. Patent Application Serial Numbers: 10/741,785 filed December 19, 2003; 60/478,763 filed December 19, 2003; 10/741,868 filed December 30, 2003. US5207644 A relates to a percutaneously implantable catheter system that has an infusion chamber with an end closed by an insertion cone, an opposite end closed by a closure plug, and a fluid reservoir therebetween, the infusion chamber wall provided with an aperture as a puncture window, through which an injection needle can be inserted into the chamber, for piercing the closure plug, the infusion chamber further comprising anchoring means, including anchoring loops and/or pins, connected to the chamber wall, where the infusion chamber, with the anchoring means pressed against the chamber wall, is accommodated in an outer casing which can be stripped off after the implantation operation to allow the anchorage means to spread out so as to anchor the infusion chamber in position. DE19745654 A1 relates to a port for subcutaneous infusion, the port comprising a housing with a filling chamber, an outlet opening and an injection opening with a self closing septum.

### Summary of the Invention

In accordance with the present invention, there is provided an implantable surgical injection port as claimed in appended claims. The injection port has an undeployed position, and a deployed position wherein it is attached to tissue. The port includes a housing having a closed distal end, an open proximal end and a fluid reservoir therebetween. The port further includes a needle penetrable septum attached to the housing about the open proximal end. The port even further includes at least one attachment mechanism mounted to the housing at a pivot point along an outer periphery of the housing. The attachment mechanism is an arcuate hook pivotable with respect to the housing, wherein the arcuate hook having a length extending at least 180° about the pivot point.

Said housing further includes at least one recessed portion at said distal end thereof for receiving said free end of said attachment mechanism when said port is in its deployed position.

Preferably the injection port further includes a catheter connection tube attached to said housing and in fluid communication with said reservoir.

Preferably said housing comprises titanium.

Preferably said septum self seals after being punctured by a needle and the needle is withdrawn.

Preferably said septum comprises silicone.

Preferably said injection port includes at least three said attachment mechanisms.

Preferably said attachment mechanisms are equally spaced along said outer periphery.

Preferably said outer periphery of said injection port is adjacent said distal end of said housing.

### Detailed Description of the Drawings

The novel features of the invention are set forth with particularity in the appended claims. The invention itself, however, both as to organization and methods of operation, together with further objects and advantages thereof, may best be understood by reference to the following description, taken in conjunction with the accompanying drawings in which:
Figure 1 is a perspective view of a surgically implantable fluid port made in accordance with the present invention, showing the port attached to an adjustable gastric band.
Figure 2 is a perspective view of a surgically implantable fluid port made in accordance with the present invention.
Figure 3 is a cross section of the port shown in Figures 1 and 2, taken along line 3-3 in Figure 1.
Figure 4 is a view similar to that of Figure 3 but showing the fluid port implanted within a patient.

### Detailed Description of the Invention

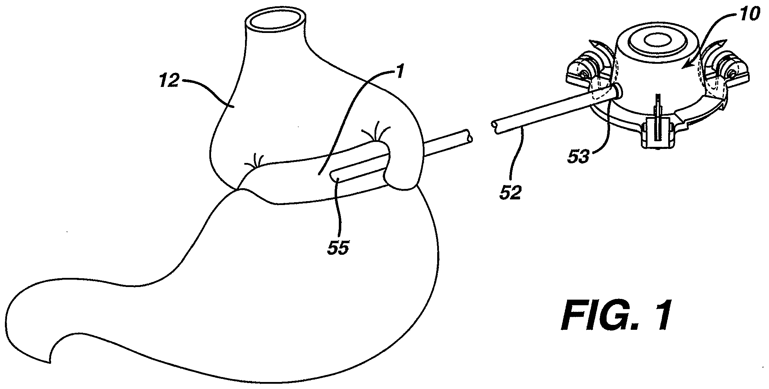

Referring now to the drawings wherein like numerals indicate the same elements throughout the views, as stated above there is shown in Figure 1 an adjustable gastric band 1 of the type described in the above mentioned references. Band 1 is implanted within a body of a patient to surround the stomach 12. The inflatable portion of the band is in fluid communication with injection port 10 via a catheter tube 52. Tube 52 has a proximal end 53 attached to the port 10 and a distal end 55 attached to adjustable gastric band 1. Port 10 can be used for a wide range of devices in the medical field and not only for gastric bands. For example the port can also used for vascular access for drug delivery.

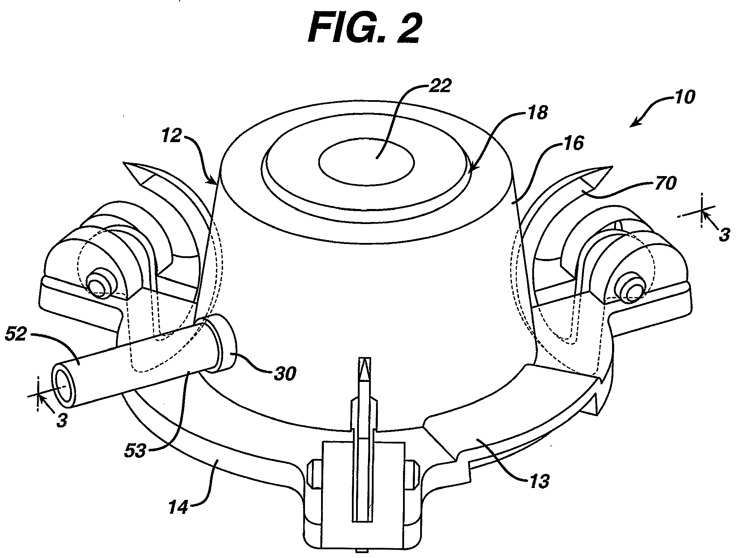

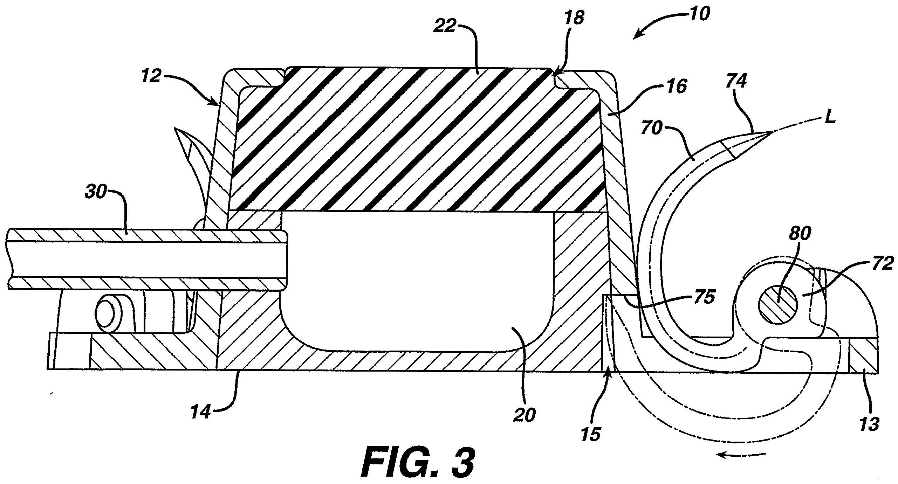

As seen from Figures 2 and 3, surgically implantable injection port 10 includes a housing 12. Housing 12 can be made from any number of materials including stainless steel, titanium, or polymeric materials. Housing 12 has a distal back portion or closed distal end 14 and a perimeter wall portion 16 extending proximally from the back portion 14 at an angle. Wall portion 16 defines a proximal opening or open proximal end 18, and a fluid reservoir 20 between opening 18 and back portion 14. The port includes a needle penetrable septum 22 attached to the housing about the opening 18 so as to cover the opening and seal the reservoir 20. Septum 22 can be made from any number of materials including silicone. Septum 22 is preferably placed in a proximal enough position such that the depth of the reservoir 20 is sufficient enough to expose the open tip of a needle, such as a Huber needle, so that fluid transfer can take place. Septum 22 is preferably arranged so that it will self seal after being punctured by a needle and the needle is withdrawn. In one embodiment, the septum is made from silicone which is under compression when attached to the housing. Port 10 further includes a catheter tube connection member 30, in fluid communication with reservoir 20.

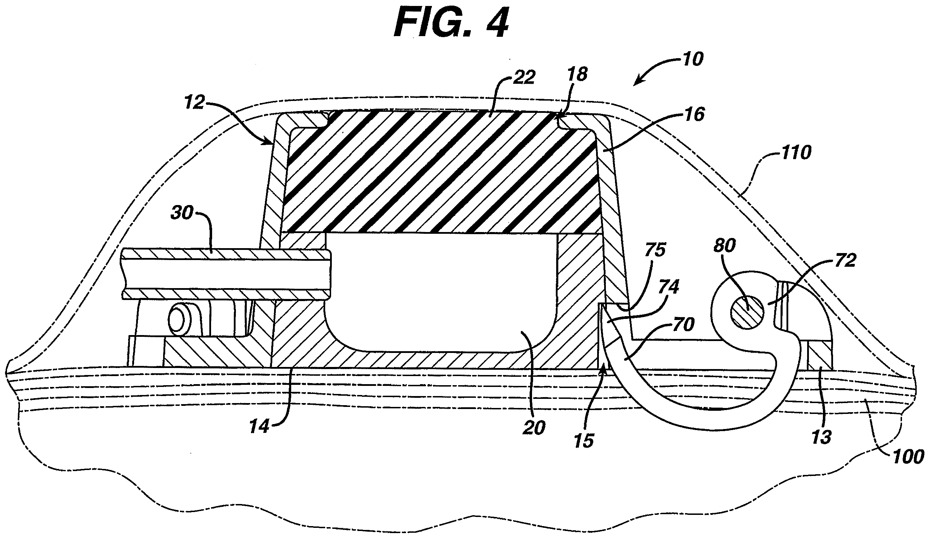

As seen from the figures, port 1 one or more attachment mechanisms 70. The figures herein show three attachment mechanisms all substantially identical and equally spaced from each other. Attachment mechanisms 70 are mounted to the housing 12 at a pivot point 80 along an outer periphery 13 of the housing 12. As seen from the figures, attachment mechanisms 70 are arcuate hooks pivotable with respect to the housing. Attachment mechanisms 70 have an arcuate length L extending at least 180° about the pivot point. Implantable surgical injection port 10 has an undeployed position, shown as a solid line in Figure 3, and a deployed position, shown as the phantom line in Figure 3 and in Figure 4, wherein the port is attached to tissue. Attachment mechanisms 70 can be made from any number of materials including stainless steel, titanium or absorbable materials such as polyglactin and poliglecaprone.

Attachment mechanism 70 has a fixed end 72 pivotally attached to the housing 12 at pivot point 80. The design allows a surgeon to use forceps and drive the fastener through the tissue until the free end 74 rests against the flat 75. In this way the patient is protected from the sharp end of the tip. Attachment mechanism 70 also includes a free end 74 which has a sharp or pointed configuration. Housing 12 further includes at least one recessed portion 15 along its distal end 14. Recessed portion 15 is designed to receive the free end 74 of attachment mechanisms 70 when the port 1 is in its deployed position. This design prevents any exposure of the sharp free end to tissue after the port has been implanted.

The above described 180° hook or attachment mechanisms provide advantages over prior 90° or less hooks. As seen from Figure 4, the above described attachment mechanism allows the hook to engage a greater area of tissue, and allows for two locking points, entry into and then out of the fascia. This provides for better sacrament of the port to the tissue. Further no "sharp" is exposed to the patient. A further advantage of the fastener configuration is that the fastener follows a constant radius when pushing through the tissue. By maintaining a constant radius the fastener never induces a compressive force onto the fascia. This should minimize pain because the fastener is not "compressing or squeezing" nerves.

In practice, the physician would create an incision in the skin 110 of a patient to expose the fascia according to well known surgical techniques. Thereafter, as seen from Figure 4, the port 1 could be placed against the fascia 100 of the patient with the port in its undeployed position. Thereafter, the physician could rotate, manually or otherwise, the attachment mechanism substantially greater than 90° and preferably at least 180° so that the hook enters and then exits the fascia. The design allows a surgeon to use forceps and drive the fastener through the tissue until the free end 74 rests against the flat 75. In this way the patient is protected from the sharp end of the tip. This could be done for each attachment mechanism on the device. Thereafter, the catheter tube 52 would be connected to connection member 30, and the patient is sewn up.

It will become readily apparent to those skilled in the art that the above invention has equally applicability to other types of implantable bands. For example, bands are used for the treatment of fecal incontinence. One such band is described in U.S. Patent 6,461,292. Bands can also be used to treat urinary incontinence. One such band is described in U.S. Patent Application 2003/0105385. Bands can also be used to treat heartburn and/or acid reflux. One such band is described in U.S. Patent 6,470,892. Bands can also be used to treat impotence. One such band is described in U.S. Patent Application 2003/0114729.

While preferred embodiments of the present invention have been shown and described herein, it will be obvious to those skilled in the art that such embodiments are provided by way of example only. Numerous variations, changes, and substitutions will now occur to those skilled in the art without departing from the invention. For example, as would be apparent to those skilled in the art, the disclosures herein have equal application in robotic-assisted surgery. In addition, it should be understood that every structure described above has a function and such structure can be referred to as a means for performing that function. Accordingly, it is intended that the invention be limited only by the scope of the appended claims.

## Claims

1. An implantable surgical injection port (10) having an undeployed position, and a deployed position wherein it is attached to tissue, said port comprising:
a) a housing (12) having a closed distal end (14), an open proximal end (18) and a fluid reservoir (20) therebetween;
b) a needle penetrable septum (22) attached to said housing about said open proximal end (18); and
c) at least one attachment mechanism (70), **characterised in that** the at least one attachment mechanism is mounted to said housing at a pivot point (80) along an outer periphery (13) of said housing, said attachment mechanism comprising an arcuate hook pivotable with respect to said housing in order to engage the hook to tissue, said arcuate hook having a sharp free end (74) and an arcuate length extending at least 180° about said pivot point, wherein said housing includes at least one recessed portion (15) at said distal end thereof for receiving the free end (74) of said attachment mechanism when said port is in its deployed position, wherein said arcuate hook is pivotable about said pivot point until said free end (74) rests against a flat (75) of the housing in said recessed portion.

2. The injection port of claim 1, further including a catheter connection tube attached to said housing and in fluid communication with said reservoir.

3. The injection port of any one of the preceding claims, wherein said housing comprises titanium.

4. The injection port of any one of the preceding claims, wherein said septum self seals after being punctured by a needle and the needle is withdrawn.

5. The injection port of any one of the preceding claims, wherein said septum comprises silicone.

6. The injection port of any one of the preceding claims, wherein said injection port includes at least three said attachment mechanisms.

7. The injection port of claim 6 wherein said attachment mechanisms are equally spaced along said outer periphery.

## Patentansprüche

1. Implantierbare chirurgische Injektionsöffnung (10), die eine nicht eingesetzte Position und eine Einsatzposition aufweist, in der sie an Gewebe befestigt ist, wobei die Öffnung das Folgende umfasst:
a) ein Gehäuse (12) mit einem geschlossenen distalen Ende (14), einem offenen proximalen Ende (18) und einem Fluidreservoir (20) dazwischen;
b) ein mit einer Nadel durchstechbares Septum (22), das am Gehäuse um das offene proximale Ende (18) befestigt ist; und
c) mindestens einen Befestigungsmechanismus (70), **dadurch gekennzeichnet, dass** der mindestens eine Befestigungsmechanismus am Gehäuse an einem Drehpunkt (80) entlang eines Außenumfangs (13) des Gehäuses angebracht ist, wobei der Befestigungsmechanismus einen bogenförmigen Haken umfasst, der bezüglich des Gehäuses schwenkbar ist, um den Haken mit Gewebe in Eingriff zu bringen, wobei der bogenförmige Haken ein spitzes freies Ende (74) und eine bogenförmige Länge aufweist, die sich mindestens 180° um den Drehpunkt erstreckt, wobei das Gehäuse mindestens einen ausgesparten Abschnitt (15) an seinem distalen Ende zur Aufnahme des freien Endes (74) des Befestigungsmechanismus in der Einsatzposition der Öffnung aufweist, wobei der bogenförmige Haken um den Drehpunkt schwenkbar ist, bis das freie Ende (74) an einer Fläche (75) des Gehäuses im ausgesparten Abschnitt anliegt.

2. Injektionsöffnung nach Anspruch 1, ferner umfassend einen Katheteranschlussschlauch, der am Gehäuse befestigt ist und mit dem Reservoir in Fluidverbindung steht.

3. Injektionsöffnung nach einem der vorhergehenden Ansprüche, wobei das Gehäuse Titan umfasst.

4. Injektionsöffnung nach einem der vorhergehenden Ansprüche, wobei das Septum sich selbst abdichtet, nachdem es von einer Nadel durchstochen wurde und die Nadel zurückgezogen wird.

5. Injektionsöffnung nach einem der vorhergehenden Ansprüche, wobei das Septum Silikon umfasst.

6. Injektionsöffnung nach einem der vorhergehenden Ansprüche, wobei die Injektionsöffnung mindestens drei solcher Befestigungsmechanismen aufweist.

7. Injektionsöffnung nach Anspruch 6, wobei die Befestigungsmechanismen in gleichmäßigen Abständen entlang des Außenumfangs angeordnet sind.

## Revendications

1. Orifice d'injection (10) chirurgical implantable présentant une position non déployée, et une position déployée dans laquelle il est fixé au tissu, ledit orifice comprenant :
a) un boîtier (12) comportant une extrémité distale (14) fermée, une extrémité proximale (18) ouverte et entre les deux un réservoir (20) à fluide ;
b) un septum (22) pouvant être pénétré par une aiguille, fixé audit boîtier autour de ladite extrémité proximale (18) ouverte ; et
c) au moins un mécanisme de fixation (70), **caractérisé en ce que** ledit mécanisme de fixation est
monté sur ledit boîtier au niveau d'un point de pivotement (80) le long de la périphérie extérieure (13) dudit boîtier, ledit mécanisme de fixation comprenant un crochet arqué pouvant pivoter par rapport audit boîtier afin de solidariser le crochet au tissu, ledit crochet arqué comportant une extrémité libre (74) pointue et une longueur en arc s'étendant sur au moins 180° autour dudit point de pivotement,
dans lequel ledit boîtier comprend au moins une partie renfoncée (15) au niveau de ladite extrémité distale de celui-ci pour recevoir l'extrémité libre (74) dudit mécanisme de fixation quand ledit orifice est dans sa position déployée,
dans lequel ledit crochet arqué peut pivoter autour dudit point de pivotement jusqu'à ce que ladite extrémité libre (74) repose contre un plat (75) du boîtier dans ladite partie renfoncée.

2. Orifice d'injection selon la revendication 1, comprenant en outre un tube de raccordement de cathéter fixé audit boîtier et en communication fluidique avec ledit réservoir.

3. Orifice d'injection selon l'une quelconque des revendications précédentes, dans lequel ledit boîtier comprend du titane.

4. Orifice d'injection selon l'une quelconque des revendications précédentes, dans lequel ledit septum rétablit automatiquement l'étanchéité après perforation par une aiguille et retrait de l'aiguille.

5. Orifice d'injection selon l'une quelconque des revendications précédentes, dans lequel ledit septum comprend de la silicone.

6. Orifice d'injection selon l'une quelconque des revendications précédentes, dans lequel ledit orifice d'injection comprend au moins trois desdits mécanismes de fixation.

7. Orifice d'injection selon la revendication 6, dans lequel lesdits mécanismes de fixation sont équidistants le long de ladite périphérie extérieure.
